Europäisches Patentamt

European Patent Office

Office européen des brevets

(11) Numéro de publication: **0 123 824**
**A2**

(12) **DEMANDE DE BREVET EUROPEEN**

(21) Numéro de dépôt: **84102177.7**

(22) Date de dépôt: **01.03.84**

(51) Int. Cl.³: **F 24 F 3/16**

(30) Priorité: **01.03.83 FR 8303354**

(43) Date de publication de la demande: **07.11.84**
**Bulletin 84/45**

(84) Etats contractants désignés: **AT BE CH DE GB IT LI NL SE**

(71) Demandeur: **MOULINEX S.A., 7 à 15, rue Jules-Ferry, F-93171 Bagnolet (FR)**

(72) Inventeur: **Vielle Dominique André Société MOULINEX, Rue Charles-Granger, F-72600 Mamers (FR)**

(74) Mandataire: **May, Hans Ulrich, Thierschstrasse 27, D-8000 München 22 (DE)**

(54) **Assainisseur d'air.**

(57) Un groupe motoventilateur (12) crée un courant d'air F qui traverse un filtre (18) et qui reçoit un apport de produit odorant émanant d'une chambre émettrice (22).

Selon l'invention, l'apport de produit odorant est réglable au moyen d'un registre (28) commandé par une manette (38).

L'invention est applicable aux assainisseurs de traitement de l'air des locaux d'habitation.

EP 0 123 824 A2

- 1 -

## Assainisseur d'air

L'invention se rapporte aux assainisseurs d'air, c'est-à-dire aux appareils, destinés notamment au traitement de l'air des locaux d'habitation, qui aspirent l'air ambiant et le restituent sous une forme assainie ou purifiée.

L'invention concerne, plus précisément, les assainisseurs d'air comprenant, dans un boîtier, un groupe motoventilateur adapté à créer un courant d'air entre des ouvertures d'entrée et de sortie d'air pratiquées dans ledit boîtier, un filtre supporté par un siège interne au boîtier et traversé par la totalité dudit courant d'air, et une chambre émettrice d'un produit actif montée sur le filtre et communiquant avec l'espace situé en aval du filtre par rapport au courant d'air.

Un assainisseur d'air de ce genre est décrit, par exemple, dans le brevet GB-A-246 261.

L'invention a pour but de permettre un réglage de l'apport de produit actif en provenance de la chambre émettrice. On sait en effet que ce produit actif peut consister en une vapeur odorante émanant d'une substance solide contenue dans la chambre, et que, l'impression olfactive en résultant pouvant être très diverse selon les

0123824

- 2 -

individus, il est par suite souhaitable de pouvoir en régler l'apport dans l'atmosphère.

Dans un assainisseur d'air selon l'invention, la chambre émettrice communique avec le courant d'air par l'intermédiaire d'un registre permettant de faire varier l'apport de produit actif dans ce courant d'air, et à cet effet l'ensemble du filtre et de la chambre émettrice est monté mobile sur le siège interne au boîtier, la partie mobile dudit registre étant formée par une paroi ajourée de la chambre émettrice, tandis que la partie fixe de ce registre est formée par une contre-paroi ajourée supportée par le boîtier.

Ainsi, un déplacement du filtre sur son siège se traduit par une variation de l'ouverture du registre.

Les caractéristiques et avantages de l'invention ressortiront d'ailleurs de la description qui va suivre, à titre d'exemple, en référence aux dessins annexés dans lesquels :

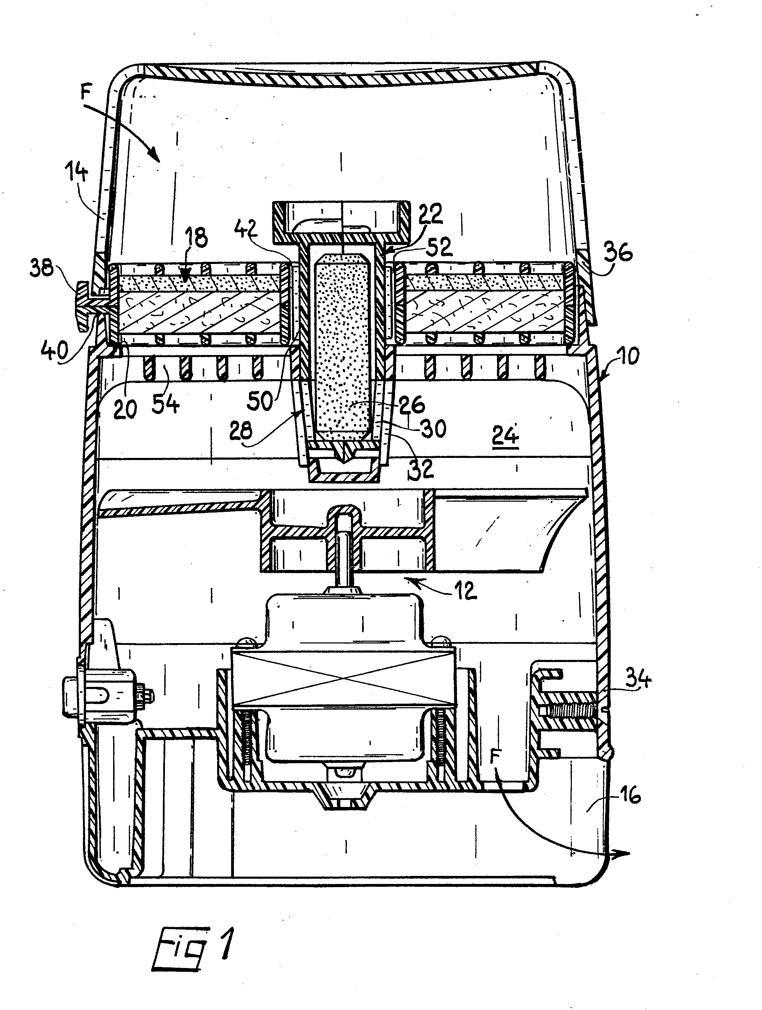

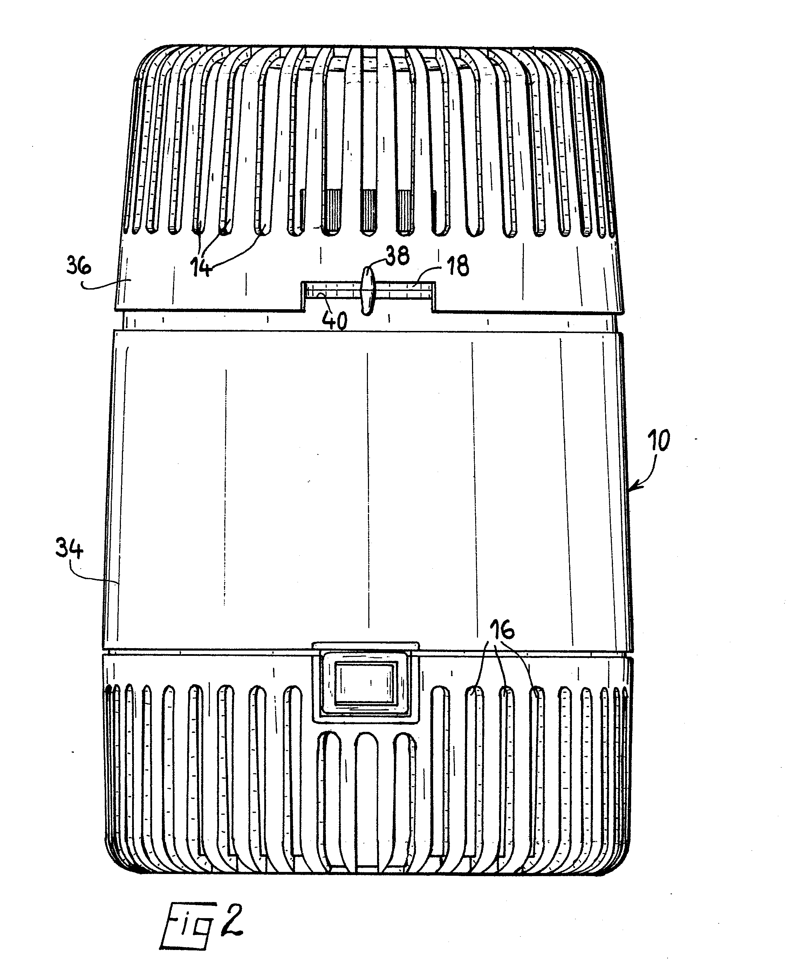

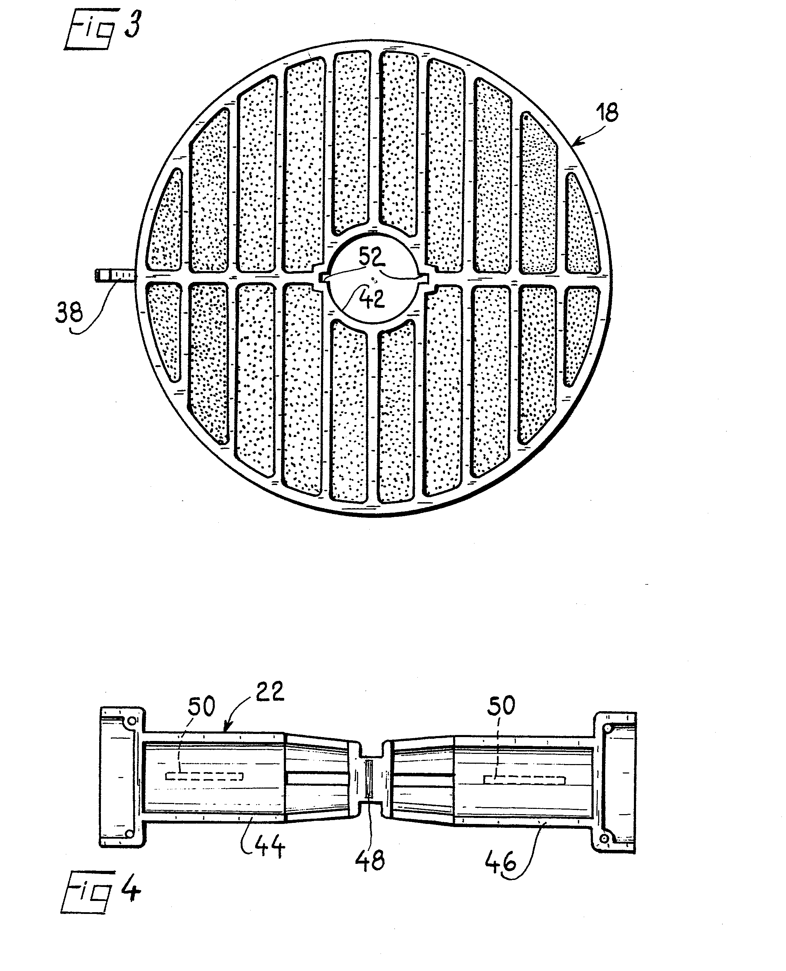

la figure 1 est une coupe verticale d'un assainisseur selon l'invention ; la figure 2 en est une vue en élévation ; la figure 3 est une vue en plan du filtre incorporé à cet assainisseur ; la figure 4 représente la chambre émettrice en position ouverte, par exemple avant qu'on y introduise la substance odorante.

L'assainisseur d'air représenté aux figures 1 et 2 comprend un boîtier 10 présentant la forme générale d'un cylindre à axe vertical. Ce boîtier contient un groupe moto-ventilateur 12 adapté à créer dans l'intérieur de ce boîtier un courant d'air de haut en bas (flèches F) entre des ouvertures d'entrée 14 et des ouvertures de sortie 16 pratiquées dans ce boîtier. Ce boîtier contient aussi un filtre 18 de retenue des impuretés, traversé par la totalité du courant d'air, et supporté par un siège 20 interne au boîtier. Sur ce filtre 18 est montée une chambre 22 qui émet une vapeur odorante et qui communique avec l'espace 24 situé en aval du filtre 18 par rapport au courant d'air. Cette vapeur odorante émane d'un bâton de substance solide 26 contenu dans la chambre 22.

Selon l'invention, la chambre émettrice 22 communique avec l'espace 24 par l'intermédiaire d'un registre 28 permettant de faire varier l'apport de produit actif dans le courant d'air. A cet effet, l'ensemble du filtre 18 et de la chambre émettrice 22 est monté mobile sur le siège 20, la partie mobile du registre étant formée par une paroi ajourée 30 de la chambre 22, tandis que la partie fixe de ce registre est formée par une contre-paroi ajourée 32 supportée par le boîtier 10.

Le boîtier cylindrique 10 est constitué en deux parties, soit une partie inférieure 34 qui constitue le réceptacle du groupe motoventilateur 12, et une partie supérieure 36 formant couvercle amovible. Les ouvertures d'entrée d'air 14 sont pratiquées dans le couvercle 36, tandis que les ouvertures de sortie d'air 16 sont pratiquées dans la partie basse du réceptacle 34.

Le filtre 18, qui comprend plusieurs couches de matériau filtrant tenues dans une armature rigide, présente une forme circulaire et est monté rotatif sur son siège 20. Ce siège est formé par une collerette qui est disposée dans un plan horizontal au voisinage de l'extrémité supérieure du réceptacle 34, et sur laquelle le filtre 18 est posé de manière amovible. Ce filtre 18 porte sur sa périphérie une manette 38 dirigée radialement vers l'extérieur, qui chevauche le bord supérieur 40 du réceptacle 34 pour saillie à l'extérieur du boîtier, et sur laquell·ager peut agir pour commander la rotation de l'en² du filtre 18 et de la chambre 22, et faire ainsi ᵥ la position du registre 28.

La char^mettrice 22 est montée de façon amovible sur le fil⫶ Elle est emmanchée dans un trou central 42 traver⫶e filtre 18 de part en part. Cette chambre présen· forme d'un tube à axe vertical qui est formé en une² pièce (fig.4) comprenant deux coquilles 44 et 46 ·assemblent l'une à l'autre selon un plan lon-gitudi²dian, et qui sont articulées l'une à l'autre en l'²ité inférieure du tube selon une ligne diamé-trale ·a face latérale externe de ce tube porte des nervu²rticales 50 qui s'engagent dans des rainures compl²aires 52 pratiquées dans la face interne du trou ·al 42 du filtre, ces nervures et rainures cons-titu²semble des moyens d'entraînement, grâce aux-quel·²ouvement de rotation du filtre 18 entraîne la rota²de la chambre 22.

La ᵨajourée 30 de la chambre 22, qui forme la par-tie ²le du registre 28, est formée par la région in-fér⫶ tronconique de la face latérale du tube, tandis que·ontre-paroi ajourée 32, qui forme la partie fixe du ⫶tre 28, présente une forme tronconique complé-mer². Comme on le voit sur la figure 1, cette contre-par² est supportée par une grille 54, formée de bar²x espacés, solidaire de la collerette 20 ; c'est-à⸗ que la contre-paroi 32 forme, en fait, une seule pi²avec le boîtier 10.

P⸗t le fonctionnement du groupe motoventilateur 12, l ambiant est aspiré dans le boîtier 10 par les ou-v⸗es d'entrée 14 et traverse ensuite le filtre 18 qui r²t au passage diverses impuretés, telles que pous-

sières et mauvaises odeurs. Dans l'espace 24 situé en aval du filtre, le courant d'air reçoit, à travers le registre 28, un apport de vapeur odorante en provenance de la chambre émettrice 22. Cet apport peut être réglé par l'usager au moyen de la manette 38 de commande du registre 28. L'air est rejeté du boîtier 10, sous une forme purifiée et parfumée, par les ouvertures de sortie 16.

Lorsque l'usager désire procéder au remplacement du filtre 18 et/ou du bâton de substance odorante 26, il enlève le couvercle 36, ce qui lui donne accès à la chambre émettrice 22 et au filtre 18. La chambre 22 peut être enlevée de l'appareil, indépendamment du filtre, ceci par simple traction de cette chambre vers le haut, avec coulissement des nervures 50 dans les rainures 52.

Revendications

1. Assainisseur d'air comprenant, dans un boîtier (10), un groupe motoventilateur (12) adapté à créer un courant d'air entre des ouvertures d'entrée et de sortie d'air (14,16) pratiquées dans ledit boîtier, un filtre (18) supporté par un siège (20) interne au boîtier (10) et traversé par la totalité du courant d'air, et une chambre émettrice d'un produit actif (22) montée sur le filtre (18) et communiquant avec l'espace (24) situé en aval du filtre (18) par rapport au courant d'air, c a r a c t é r i s é  en ce que, la chambre émettrice (22) communiquant avec le courant d'air par l'intermédiaire d'un registre (28) permettant de faire varier l' apport de produit actif dans ce courant d'air, l'ensemble du filtre (18) et de la chambre émettrice (22) est à cet effet monté mobile sur ledit siège (20) interne au boîtier, la partie mobile dudit registre (28) étant formée par une paroi ajourée (30) de la chambre émettrice (22), tandis que la partie fixe de ce registre (28) est formée par une contre-paroi ajourée (32) supportée par le boîtier (10).

2. Assainisseur d'air selon la revendication 1, c a r a c t é r i s é  en ce que le mouvement de l'ensemble du filtre (18) et de la chambre émettrice (22) est commandé par action de l'usager sur une manette (38) portée par le filtre (18) et saillante hors du boîtier(10).

3. Assainisseur d'air selon la revendication 2, c a r a c t é r i s é  en ce que le filtre (18) est circulaire et est monté rotatif sur le siège (20), la manette (38) étant portée par la périphérie du filtre (18) et dirigée radialement vers l'extérieur.

4. Assainisseur d'air selon l'une quelconque des revendications précédentes,
c a r a c t é r i s é  en ce que la chambre émettrice
(22) est montée de façon amovible sur le filtre (18).

5. Assainisseur d'air selon les revendications 3 et 4,
c a r a c t é r i s é  en ce que la chambre émettrice
(22) est montée dans un trou central (42) traversant le
filtre (18) de part en part, des moyens d'entraînement
en rotation (50,52) étant agencés entre la face interne de ce
trou (42) et la face externe de la chambre (22).

6. Assainisseur d'air selon la revendication 5,
c a r a c t é r i s é  en ce que, le boîtier (10) présentant la forme générale d'un cylindre à axe vertical
traversé de haut en bas par le courant d'air, la partie
inférieure (34) de ce cylindre constitue le réceptacle
du groupe motoventilateur (12), le siège (20) du filtre
(18) est formé par une collerette qui est disposée dans
un plan horizontal au voisinage de l'extrémité supérieure de ce réceptacle (34) et sur laquelle le filtre (18)
est posé de façon amovible, la manette de commande (38)
chevauche le bord supérieur (40) de ce réceptacle, et
la partie supérieure du cylindre constitue un couvercle
(36) qui est monté amovible pour laisser accès à la
chambre émettrice (22) et au filtre (18), les ouvertures
(14,16) d'entrée et de sortie d'air étant pratiquées
respectivement dans ce couvercle (36) et dans la partie
basse du réceptacle (34).

7. Assainisseur d'air selon la revendication 6,
c a r a c t é r i s é  en ce que la contre-paroi ajourée
(32), qui forme la partie fixe du registre (28), est supportée par une grille (54) solidaire du siège (20).

0123824

8. Assainisseur d'air selon la revendication 7, c a r a c t é r i s é en ce que la chambre émettrice (22) présente la forme d'un tube à axe vertical dont la face latérale externe porte des nervures verticales (50) qui forment partie des moyens d'entraînement et qui s'engagent à cet effet dans des rainures complémentaires (52) pratiquées dans la face interne du trou central (42) du filtre (18), tandis que la région inférieure de ce tube est ajourée pour former la partie mobile (30) du registre (28).

9. Assainisseur d'air selon la revendication 8, c a r a c t é r i s é en ce que la région inférieure ajourée (30) est une zone tronconique de la face latérale du tube, tandis que la contre-paroi fixe (32) présente une forme tronconique complémentaire.

10. Assainisseur d'air selon la revendication 8 ou la revendication 9, c a r a c t é r i s é en ce que le tube est formé en une seule pièce comprenant deux coquilles (44,46) assemblées l'une à l'autre selon un plan longitudinal médian et articulées en une extrémité du tube selon une ligne diamétrale (48).

Fig 1

Fig 2

*Fig* 3

*Fig* 4